Europäisches Patentamt

(19) European Patent Office    (11) Publication number: **0 026 245**

Office européen des brevets    **B1**

(12)    **EUROPEAN PATENT SPECIFICATION**

(45) Date of publication of patent specification: **16.05.84**    (51) Int. Cl.³: **C 07 C 51/27, C 07 C 63/00**

(21) Application number: **79302004.1**

(22) Date of filing: **26.09.79**

(54) **Preparation of a mixture of polycyclic aromatic polycarboxylic acids soluble in acetone and in water, and mixture obtained thereby.**

(43) Date of publication of application:
**08.04.81 Bulletin 81/14**

(45) Publication of the grant of the patent:
**16.05.84 Bulletin 84/20**

(84) Designated Contracting States:
**AT BE CH DE FR GB IT LU NL SE**

(56) References cited:
**DE-C- 743 225**
**GB-A- 797 726**
**US-A-2 555 410**
**US-A-3 209 024**
**US-A-4 137 418**

(73) Proprietor: **GULF RESEARCH & DEVELOPMENT COMPANY**
**P.O. Box 2038**
**Pittsburgh Pennsylvania 15230 (US)**

(72) Inventor: **Schulz, Johann Gustav D.**
**201 Conover Road**
**Pittsburgh, Pennsylvania 15208 (US)**
Inventor: **Sabourin, Edward T.**
**4324 Winchester Drive**
**Allison Park, Pennsylvania 15101 (US)**

(74) Representative: **Huskisson, Frank Mackie**
**FITZPATRICKS 4 West Regent Street**
**Glasgow G2 1RS Scotland (GB)**

Courier Press, Leamington Spa, England.

## Description

### 1. Field of the invention

This invention relates to a process for preparing a mixture of polycyclic aromatic polycarboxylic acids that is substantially soluble in acetone and substantially soluble in water which comprises subjecting a slurry containing coal to reaction with aqueous nitric acid in an atmosphere containing molecular oxygen, mechanically separating the solids in the resulting slurry, separating nitric acid and water from the resulting filtrate and then extracting the remainder of said filtrate with a polar solvent to obtain said mixture of polycyclic aromatic polycarboxylic acids substantially soluble in acetone and substantially soluble in water.

### 2. Description of the prior art

In our U.S. Patent No. 4,137,418 entitled Organic Acids and Process for Preparing Same, we disclosed a process for preparing a mixture of polycyclic aromatic polycarboxylic acids that is substantially soluble in acetone and substantially soluble in water which involved subjecting a slurry containing coal to reaction with aqueous nitric acid, mechanically separating the solids in the resulting slurry, separating nitric acid and water from the resulting filtrate and then extracting the remainder of said filtrate with a polar solvent to obtain a said mixture of polycyclic aromatic polycarboxylic acids substantially soluble in acetone and substantially soluble in water. There is disclosed in US—A— 2555410 a process for making organic acids from carbonaceous material. Of specific interest in that disclosure is the production of *mono*-aromatic benzene compounds such as mellitic, benzenepenta-carboxylic acid and the di-, tri- and tetra-acids, and also aliphatic acids such as oxalic and acetic acids. The process involves reacting coal with nitric acid in the presence of excess oxygen. The desired acids can be recovered by extraction with an appropriate organic solvent.

DE—C—743,225 also discloses in general terms the nitric acid oxidation of coal and coal derivatives to give water-soluble organic acids.

We have now found, unexpectedly, that we can greatly increase the amount of acetone-soluble, water-soluble polycyclic aromatic polycarboxylic acids obtained in the process disclosed and claimed in said U.S. Patent No. 4,137,418 if we carry out the nitric acid reaction therein in an atmosphere containing molecular oxygen.

The individual components of the acetone-soluble, water-soluble polycyclic aromatic polycarboxylic acids obtained herein are believed to be composed of condensed and/or non-condensed benzene rings, with an average number of benzene rings in the individual molecules ranging from one to three, but generally from one to two. On the average, the number of carboxyl groups carried by the individual molecules are believed to range from two to eight, generally from two to five, and the average number of nitro groups from 0 to four, generally from 0 to two. The average molecular weight of the mixture is believed to range from 200 to 500, generally from 300 to 400, and the average neutral equivalent will range from 50 to 200, generally from 70 to 120. A typical analysis of the desired mixture is defined below in Table I in approximate amounts.

TABLE I

|  | Weight per cent Broad range | General range |
|---|---|---|
| Carbon | 35 to 60 | 37 to 48 |
| Hydrogen | 1 to 5 | 3 to 4 |
| Nitrogen | 1 to 6 | 4 to 5 |
| Oxygen | 35 to 60 | 40 to 55 |
| Sulfur | 0.1 to 0.4 | 0.1 to 0.3 |
| Ash | 0.1 to 5 | 0.1 to 2 |

In a preferred procedure herein there is introduced into a reactor an aqueous solution of nitric acid and a carbonaceous material. The nitric acid can have a concentration of from five to 90 per cent, but preferably will be in the range of from 10 to 70 per cent. The carbonaceous material is preferably a solid in the form of a slurry, for example, an aqueous slurry containing the carbonaceous material in particulate form and from 50 to 90 weight per cent of water.

The solid carbonaceous material that can be used herein can have the following composition on a moisture-free basis:

2

# O 026 245

## TABLE II

|  | Weight per cent | |
|  | Broad range | Preferred range |
| --- | --- | --- |
| Carbon | 45—95 | 60—92 |
| Hydrogen | 2.5—7 | 4—6 |
| Oxygen | 2.0—45 | 3—25 |
| Nitrogen | 0.75—2.5 | 0.75—2.5 |
| Sulfur | 0.3—10 | 0.5—6 |

The carbon and hydrogen content of the carbonaceous material will reside primarily in multi-ring aromatic compounds (condensed and/or uncondensed), heterocylic compounds, etc. Oxygen and nitrogen are believed to be present primarily in chemical combination. Some of the sulfur is believed to be present in chemical combination with the aromatic compounds and some in chemical combination with inorganic elements associated therewith, for example, iron and calcium.

In addition to the above the solid carbonaceous material being treated herein will also contain solid, primarily inorganic, compounds which will not be converted to the desired organic mixture claimed herein, which are termed ash, and are composed chiefly of compounds of silicon, aluminum, iron and calcium, with smaller amounts of compounds of magnesium, titanium, sodium and potassium. The ash content of the carbonaceous material treated herein will amount to less than about 50 weight per cent, based on the moisture-free carbonaceous material, but, in general, will amount to from 0.1 to 30 weight per cent, usually from 0.5 to 20 weight per cent.

Anthracitic, bituminous and subbituminous coal, lignitic materials, and other type of coal products referred to in ASTM D-388 are exemplary of the solid carbonaceous materials which can be treated in accordance with the process defined herein to produce the claimed organic mixture. Some of these carbonaceous materials in their raw state will contain relatively large amounts of water. These can be dried prior to use herein. The carbonaceous material, prior to use, is preferably ground in a suitable attrition machine, such as a hammermill, to a size such that at least about 50 per cent of the carbonaceous material will pass through a 40-mesh (U.S. Series) sieve. As noted, the carbonaceous material is slurried in a suitable carrier, preferably water, prior to reaction with nitric acid. If desired, the carbonaceous material can be treated, prior to reaction herein, using any conventional means, to remove therefrom any materials forming a part thereof that will not be converted in reaction with nitric acid herein.

The reactant mixture in the reactor is stirred while being maintained at a temperature of from 15° to 200°C., preferably from 50 to 120°C., and a molecular oxygen pressure of from 98 kPa to 9.8 MPa, preferably from 490 kPa to 4.9 MPa for from 0.5 to 15 hours, preferably from two to six hours. In order to obtain the desired mixture herein without losing appreciable amounts of carboxyl groups on the acids that are formed during the oxidation, and to obtain the desired acids in high yields in the reactor, it is absolutely critical that the reaction conditions therein, namely nitric acid concentration, temperature, pressure of molecular oxygen and reaction time, be so correlated to minimize and, preferably, to avoid decarboxylation. Gaseous products, such as nitrogen oxides, if any, can be removed from the reaction zone.

The reaction product is removed from the reactor in any convenient manner. We have found that the reaction product is soluble in, or reactable with, sodium hydroxide. At this point it is necessary to separate the oxidized product from the water and nitric acid associated therewith. This separation must be accomplished in a manner so that the carboxyl groups are not removed from the acid product. Distillation for the removal of water will not suffice, because under the conditions required for such separation, a significant loss of carboxyl groups would occur. Accordingly, we have found that a mechanical separation will suffice. The reaction product is therefore led to a first separator, which can be, for example, a filter or a centrifuge.

The solids that are recovered in the first separator, also soluble in sodium hydroxide, are led to a second separator wherein they are subjected to extraction with acetone that is introduced therein. Such separation can be carried out at a temperature of from 20° to 60°C., preferably from 25° to 50°C., and a pressure of from atmospheric to 3.4 MPa preferably from atmospheric to 686 kPa. The solid material, insoluble in acetone, is removed from the second separator by one line and the acetone solution of the acid mixture by another line. The acetone solution can then be led to a drier or evaporator wherein acetone is separated therefrom by one line and an acetone-soluble, water-insoluble polyaromatic, polycarboxylic acid mixture is recovered by another line. As before, the acid mixture in the drier can be treated by so correlating the conditions therein to remove acetone therefrom in such manner so as to minimize and, preferably, avoid, decarboxylation. The temperature can be in the range of from 10° to 60°C., preferably from 20° to 50°C., the pressure from 10 millimeters of mercury to atmospheric, preferably from 30 millimeters of mercury to atmospheric, for from 0.5 to 24 hours, preferably from one to five hours.

The filtrate obtained in the first separator is removed therefrom and in all cases will contain water, nitric acid and most of the inorganic material (ash) that was present in the carbonaceous charge. Additionally, it contains the desired acetone-soluble, water-soluble organic acids desired herein.

Separation of the filtrate into its component parts can be effected as follows. It can be passed to a distillation tower maintained at a temperature of from 50° to 100°C., preferably from 70° to 90°C. and a pressure of from 10 millimeters of mercury to atmospheric, preferably from 30 millimeters of mercury to atmospheric. Under these conditions nitric acid and water are removed from the distillation tower by one line and solids by another line. The solids are led to a third separator where they are subjected to extraction with acetone introduced therein. The conditions in the third separator are similar to those used in the second separator. A mixture of the acetone-soluble, water-soluble organic acids desired herein is removed from the third separator by one line and substantially all of the inorganic material that was present in the carbonaceous charge by another line.

Several runs were carried out in which a North Dakota Lignite analyzing as follows, on a substantially moisture-free basis, was subjected to oxidation: 65.03 weight per cent carbon, 4.0 weight per cent hydrogen, 27.0 weight per cent oxygen, 0.92 weight per cent sulfur, 0.42 weight per cent nitrogen and 0.04 weight per cent moisture. The ash was further analyzed and found to contain 43 weight per cent oxygen, 7.8 weight per cent sulfur and the remainder metals. In each of Runs Nos. 1, 2 and 3, 70 per cent aqueous nitric acid was gradually added over a period of two hours to a stirred slurry maintained at 70°C. containing 800 grams of powdered lignite defined above (corresponding to 540 grams of moisture-free feed) and 600 grams of water. In each of these runs the pressure in the closed reactor at the beginning was atmospheric and was permitted to rise during the course of the reaction to its autogeneous pressure. In Run No. 4, otherwise similar to Runs Nos. 1, 2 and 3, molecular oxygen was also continuously introduced into the reactor to maintain therein a pressure of 3.3 MPa over the course of the reaction.

At the end of the reaction period the product slurry was withdrawn from the reaction zone and filtered to obtain a solids fraction and a filtrate. The solids were extracted with acetone at atmospheric temperature and pressure. The acetone solution was then subjected to evaporation at atmospheric temperature and pressure to obtain the desired mixture herein. The acetone insoluble portion was found to be soluble in sodium hydroxide and to comprise organic acids of a relatively higher molecular weight then the acetone-soluble portion.

The work-up of the filtrate was carried out as follows. Initially the filtrate was subjected to distillation to separate unreacted nitric acid and water therefrom. The remaining solids were subjected to extraction with acetone at atmospheric temperature and atmospheric pressure. The acetone solution was dried to remove acetone therefrom, resulting in the recovery of small amounts of the acetone-soluble, water-soluble organic acids substantially completely soluble in sodium hydroxide. The residue was mainly ash. The data obtained are summarized below in Table III.

### TABLE III
Weight distribution of polycyclic aromatic polycarboxylic acids

| Run no. | Total nitric acid added, grams* | Acetone-insoluble Water-insoluble, acids, grams | Acetone-soluble, water-insoluble, acids, grams | Acetone-soluble water-soluble, acids, grams | Total aromatic acids produced, grams | Total nitric acid consumed grams |
|---------|---------|---------|---------|---------|---------|---------|
| 1 | 600 | 302.9 | 142.5 | 104.0 | 549.4 | 566.9 |
| 2 | 1200 | 107.0 | 156.0 | 174.0 | 437.0 | 872.0 |
| 3 | 1800 | 86.0 | 109.0 | 200.0 | 395.0 | 1050.0 |
| 4 | 600 | 154.7 | 224.7 | 192.0 | 571.4 | 453.0 |

*On a 100 per cent basis

The results obtained above are most unusual. Doubling the amount of nitric acid in Run No. 2 over that use in Run No. 1 might have led one to expect an increase in the amount of acetone-soluble, water-soluble acids obtained, since the latter are believed to be a higher oxidation species of the other acids produced therein. Although there was an increase in the amount of acetone-soluble, water-soluble acids obtained in Run No. 2 over the amount obtained in Run No. 1, this was done at the expense of a greatly increased consumption of nitric acid. Further increase in the amount of nitric acid employed in Run No. 3 still further increased the amount of acetone-soluble, water-soluble acids obtained but, again, at the expense of a still further increase in nitric acid consumption. However, when Run No. 1 was repeated, except that the process was conducted in an atmosphere containing molecular oxygen, the amount of acetone-soluble, water-soluble acids obtained was about the same as in Run No. 3 but with less than half the consumption of nitric acid.

Although we have stated above that the desired composition is acetone-soluble and we have shown the use of acetone as suitable in the process defined herein, this has been done merely as a characterization of the composition and to exemplify one embodiment of our process. Many polar solvents alone, or polar solvents in combination with other solvents, can be used in place of acetone herein. Among the polar solvents, or combination of solvents, that can be used are methanol, ethanol, isopropanol, methyl ethyl ketone, tetrahydrofuran, dioxane, cyclohexanone, tetrahydrofurfuryl alcohol, acetone in combination with methanol, methyl ethyl ketone in combination with methanol, isopropanol or water, tetrahydrofuran in combination with methanol or water, dioxane in combination with methanol or water, etc.

**Claims**

1. A process for the oxidation of a carbonaceous material to obtain a mixture of polycyclic aromatic polycarboxylic acids which is soluble in acetone and water comprising oxidising coal with nitric acid in the presence of molecular oxygen characterised in that coal is slurried with water, the slurry so formed is reacted with nitric acid to oxidise the coal to give a mixture of oxidation product and solid residue, the solids, water and nitric acid are removed from the oxidation product to recover an acid mixture the said acid mixture is extracted with a polar solvent and a fraction of the said acid mixture is recovered from the solvent extract to yield a mixture of polycyclic aromatic polycarboxylic acids which are substantially soluble in acetone and in water.

2. A process as claimed in claim 1 in which the slurry contains from 50 to 90 percent by weight of water.

3. A process as claimed in claim 1 or claim 2 wherein said polar solvent is acetone.

4. A process as claimed in claim 1 or 2 or 3 wherein the nitric acid has a concentration of from 5 to 90 percent and the reaction is carried out at a temperature of from 15° to 200°C and a pressure of molecular oxygen of from 98 kPa to 9.8 MPa for from 0.5 to 15 hours.

5. A process as claimed in claim 4 wherein the nitric acid has a concentration of from 10 to 70 percent and the reaction is carried out at a temperature of from 50°C to 120°C and a pressure of molecular oxygen of from 490 kPa to 4.9 MPa for from two to six hours.

6. A process as claimed in any preceding claim wherein the solids are removed from the oxidation product by filtration.

7. A process as claimed in claim 6 wherein the separation of nitric acid and water are removed from the filtrate is effected by distillation.

8. A process as claimed in any preceding claim wherein the coal is lignite.

9. A mixture of polycyclic aromatic polycarboxylic acids substantially soluble in acetone and substantially soluble in water resulting from the process of claim 1.

**Patentansprüche**

1. Ein Verfahren zur Oxydation eines kohlenstoffhaltigen Materials zur Gewinnung eines Gemisches von polycyclischen aromatischen Polycarbonsäuren, das in Aceton und in Wasser löslich ist, umfassend das Oxydieren von Kohlen mit Salpetersäure in Gegenwart von molekularem Sauerstoff, dadurch gekennzeichnet, daß Kohlen mit wasser aufgeschlämmt wird, die solcherart gebildete Aufschlämmung mit Salpetersäure umgesetzt wird, um die Kohle zu oxydieren und ein Gemisch aus Oxydationsprodukt und festem Rückstand zu ergeben, daß die Feststoffe, Wasser und Salpetersäure von dem Oxydationsprodukt abgetrennt werden, um ein Säuergemisch zu ergeben, daß das genannte Säuregemisch mit einem polaren Lösungsmittel extrahiert wird und daß ein Teil des genannten Säuregemisches aus dem Lösungsmittelextrakt gewonnen wird, um ein Gemisch von polycyclischen aromatischen Polycarbonsäuren zu ergeben, die im wesentlichen in Aceton und in Wasser löslich sind.

2. Ein Verfahren nach Anspruch 1, worin die Aufschlämmung von 50 bis 90 Gew.-% Wasser enthält.

3. Ein Verfahren nach Anspruch 1 oder 2, worin das genannte polare Lösungsmittel Aceton ist.

4. Ein Verfahren nach Anspruch 1, 2 oder 3, worin die Salpetersäure eine Konzentration von 5 bis 90% aufweist und die Umsetzung bei einer Temperatur von 15 bis 200°C und einem Druck des molekularen Sauerstoffs von 98 kPa bis 9,8 MPa während 0,5 bis 15 h ausgeführt wird.

5. Ein Verfahren nach Anspruch 4, worin die Salpetersäure eine Konzentration von 10 bis 70% aufweist und die Reaktion bei einer Temperatur von 50 bis 120°C und einem Druck des molekularen Sauerstoffs von 490 kPa bis 4,9 MPa während 2 bis 6 h ausgeführt wird.

6. Ein Verfahren gemäß einem der vorstehenden Ansprüche, worin die Feststoffe von dem Oxydationsprodukt durch Filtration abgetrennt werden.

7. Ein Verfahren nach Anspruch 6, worin die Abtrennung von Salpetersäure und Wasser von dem Filtrat durch Destillation bewirkt wird.

8. Ein Verfahren nach einem der vorstehenden Ansprüche, worin die Kohle Lignit ist.

9. Ein Gemisch von polycyclischen aromatischen Polycarbonsäuren, die im wesentlichen in Aceton und im wesentlichen in Wasser löslich sind, als Ergebnis aus dem Verfahren von Anspruch 1.

**Revendications**

1. Procédé d'oxydation d'une matière carbonée en vue d'obtenir un mélange d'acides polycarboxyliques aromatiques polycycliques, ce mélange étant soluble dans l'acétone et l'eau, ce procédé consistant à oxyder du charbon avec de l'acide nitrique en présence d'oxygène moléculaire, caractérisé en ce qu'on forme un bouillie du charbon avec de l'eau, on fait réagir la bouillie ainsi formée avec de l'acide nitrique pour oxyder le charbon et obtenir un mélange d'un produit d'oxydation et d'un résidu solide, on élimine les solides, l'eau et l'acide nitrique du produit d'oxydation pour récupérer un mélange acide, on extrait ce mélange acide avec un solvant polaire et, du produit d'extraction par solvant, on récupère une fraction de ce mélange acide pour obtenir un mélange d'acides polycarboxyliques aromatiques polycycliques, ce mélange étant pratiquement soluble dans l'acétone et l'eau.

2. Procédé suivant la revendication 1, caractérisé en ce que la bouillie contient 50 à 90% en poids d'eau.

3. Procédé suivant la revendication 1 ou 2, caractérisé en ce que le solvant polaire est l'acétone.

4. Procédé suivant la revendication 1, 2 ou 3, caractérisé en ce que l'acide nitrique a une concentration de 5 à 90%, tandis que l'on effectue la réaction à une température comprise entre 15 et 200°C et sous une pression d'oxygène moléculaire se situant entre 98 kPa et 9,8 MPa pendant une période de 0,5 à 15 heures.

5. Procédé suivant la revendication 4, caractérisé en ce que l'acide nitrique a une concentration de 10 à 70%, tandis que l'on effectue la réaction à une température se situant entre 50 et 120°C et sous une pression d'oxygène moléculaire se situant entre 490 kPa et 4,9 MPa pendant une période de 2 à 6 heures.

6. Procédé suivant l'une quelconque des revendications précédentes, caractérisé en ce qu'on élimine les solides du produit d'oxydation par filtration.

7. Procédé suivant la revendication 6, caractérisé en ce que la séparation de l'acide nitrique et de l'eau éliminés du filtrat est effectuée par distillation.

8. Procédé suivant l'une quelconque des revendications précédentes, caractérisé en ce que le charbon est la lignite.

9. Mélange d'acides polycarboxyliques aromatiques polycycliques, ce mélange étant pratiquement soluble dans l'acétone et pratiquement soluble dans l'eau, résultant du procédé suivant la revendication 1.